(19) Europäisches Patentamt
European Patent Office
Office européen des brevets

(11) **EP 3 230 393 B1**

(12) **EUROPEAN PATENT SPECIFICATION**

(45) Date of publication and mention of the grant of the patent:
**24.04.2019 Bulletin 2019/17**

(21) Application number: **15832820.3**

(22) Date of filing: **11.12.2015**

(51) Int Cl.:
*C09D 133/26* (2006.01)          *C08F 220/40* (2006.01)
*C08F 220/54* (2006.01)          *C08F 220/56* (2006.01)
*C08F 230/08* (2006.01)          *C09D 143/04* (2006.01)

(86) International application number:
**PCT/IB2015/002470**

(87) International publication number:
**WO 2016/092372 (16.06.2016 Gazette 2016/24)**

(54) **NEW CLICKABLE POLYMERS AND GELS FOR MICROARRAY AND OTHER APPLICATIONS**

NEUE CLICKBARE POLYMERE UND GELE FÜR MICROARRAY UND ANDERE ANWENDUNGEN

NOUVEAUX POLYMERES CLICABLES ET GELS POUR PUCES ET AUTRES APPLICATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.12.2014 US 201462091362 P**

(43) Date of publication of application:
**18.10.2017 Bulletin 2017/42**

(73) Proprietor: **Chiari, Marcella**
**20131 Milano (IT)**

(72) Inventor: **Chiari, Marcella**
**20131 Milano (IT)**

(74) Representative: **Lahrtz, Fritz**
**Patentanwälte**
**Isenbruck Bösl Hörschler PartG mbB**
**Prinzregentenstraße 68**
**81675 München (DE)**

(56) References cited:
WO-A1-2011/124715          WO-A1-2014/100069
WO-A2-2014/089247          JP-A- 2012 201 634
US-A1- 2013 171 461

**Description**

BACKGROUND

The use of high-throughput microarrays is gaining increasing acceptance as a method for the screening of libraries of biomolecules, such as DNA, proteins, peptides and sugars (1-4). One of the key factors affecting the efficiency and specificity of a microarray experiment is the method used to attach the probes to the solid support. During the last decade click chemistry became a very efficient and cost-effective method of molecule immobilization (5). The basic foundations of click chemistry were developed by Sharpless et al. (5,7). To date, various modifications of this reaction are known (8). and JP-A-2012/201634, US-A-2013/171461, WO-A-100069 and WO-A-2014/089247.

However, the most suitable variant for microarray construction seems to be the copper (Cu(I))-catalyzed variant of alkyne-azide cycloaddition (CuAAC) (9,10). Typically, this cycloaddition reaction is simple and easy to perform and is compatible with many functional groups. This has been demonstrated by the covalent and orthogonal attachment of biomolecules to solid surfaces to fairly rapidly prepare microarrays (11-13).

Surface chemistries for solid phase assays can be categorized into mono-, two- or three dimensional, based on their architectures (14). Strategies enabling a distribution of immobilization points within the thickness of the coating are generally called "three-dimensional" (3D) coatings and are known to produce better signal-to-noise ratios, and wider dynamic ranges through a unique combination of characteristics that include low non-specific binding and high probe loading capacity (15).

A need exists for better arrays and materials to prepare the arrays, wherein high sensitivity, superior signal-to-noise ratio, and good yields can be achieved. 3D coatings enabling the attachment via click chemistry of biomolecules (e.g., peptides and proteins, nucleic acid compounds such as DNA and RNA, lipids, and carbohydrates such as glycans) in a functionally active form with proper orientation satify this need and have the potentiality of finding wide application in microarray technology.

In addition, needs exist to discover better materials to enable separations, including electrophoretic separations.

SUMMARY

Embodiments described herein include compositions and polymers, and methods of making and using such compositions and polymers including arrays.

One lead aspect provides for a composition comprising at least one polymer, wherein the polymer comprises a polymeric backbone comprising (or consisting essentially of or consisting of) at least three monomeric repeat units A, B, and C which are different from each other, wherein monomeric repeat unit C comprises at least one side group which comprises at least one optionally protected alkynyl group. This polymer can be uncrosslinked or crosslinked. This polymer, in one embodiment, can be part of a multilayer. This polymer, in one embodiment, can be reacted to form a gel or a hydrogel.

Another lead aspect is a composition comprising at least one polymer, wherein the polymer comprises a polymeric backbone comprising (or consisting essentially of or consisting of) at least two monomeric repeat units A and C which are different from each other, and optionally comprising at least a third monomeric repeat unit B, wherein monomeric repeat unit A is a substituted acrylamide monomeric repeat unit, and monomeric repeat unit C comprises at least one side group which comprises at least one optionally protected alkynyl group. This polymer can be uncrosslinked or crosslinked. This polymer, in one embodiment, can be part of a multilayer. This polymer, in one embodiment, can be reacted to form a gel or a hydrogel.

Still another lead aspect is a composition comprising at least one polymer, wherein the polymer comprises a polymeric backbone comprising (or consisting essentially of or consisting of) at least two monomeric repeat units B and C which are different from each other, and optionally comprising at least a third monomeric repeat unit A, wherein monomeric repeat unit B comprises a silane monomeric repeat unit, and monomeric repeat unit C comprises at least one side group which comprises at least one optionally protected alkynyl group. This polymer can be uncrosslinked or crosslinked. This polymer, in one embodiment, can be part of a multilayer. This polymer, in one embodiment, can be reacted to form a gel or a hydrogel.

In one embodiment, the monomeric repeat unit C comprises at least one side group which comprises at least one optionally protected alkynyl group, wherein the polymer is formed by a functionalization reaction of a pre-polymer to form the optionally protected alkynyl group.

In one embodiment, the optionally protected alkynyl group is an unprotected alkynyl group. In another embodiment, the optionally protected alkynyl group is an unprotected alkynyl group represented by - C = C - H.

In one embodiment, the monomeric repeat unit C comprises at least one side group which comprises at least one optionally protected alkynyl group, wherein the optionally protected alkynyl group is represented by $-NH-CH_2-C \equiv CH$, dibenzocyclooctyne-amine, or dibenzocyclooctyne-PEG-amine (wherein PEG is poly(ethylene glycol). In one embodiment, the optionally protected alkynyl group is a strained cyclooctyne group.

In one embodiment, the optionally protected alkynyl group is protected. In one embodiment, the optionally protected alkynyl group is protected by a silane group. In one embodiment, the optionally protected alkynyl group is protected and represented by - C ≡ C - Si(R)$_3$, wherein R is a C$_1$-C$_{12}$ alkyl group. In one embodiment, the optionally protected alkynyl group is protected and represented by - C ≡ C - Si(CH$_3$)$_3$.

In one embodiment, the monomeric repeat unit A comprises at least one N,N-substituted acrylamide repeat unit and monomer B comprises at least one silane reactive side group. In one embodiment, the polymer comprises an all carbon backbone. In one embodiment, the monomeric repeat units are distributed randomly.

In one embodiment, the polymer consists essentially of a polymeric backbone comprising (or consisting essentially of) at least three monomeric repeat units A, B, and C which are different from each other, wherein monomeric repeat unit C comprises (or consists essentially of) at least one side group which comprises (or consists essentially of) at least one optionally protected alkynyl group. This polymer can be uncrosslinked or crosslinked. This polymer, in one embodiment, can be part of a multilayer. This polymer, in one embodiment, can be reacted to form a gel or a hydrogel.

In one embodiment, the polymer consists essentially of a polymeric backbone comprising (or consisting essentially of) at least two monomeric repeat units A and C which are different from each other, and optionally comprising (or consisting essentially of) at least a third monomeric repeat unit B, wherein monomeric repeat unit A is a substituted acrylamide monomeric repeat unit, and monomeric repeat unit C comprises (or consists essentially of) at least one side group which comprises (or consists essentially of) at least one optionally protected alkynyl group. This polymer can be uncrosslinked or crosslinked. This polymer, in one embodiment, can be part of a multilayer. This polymer, in one embodiment, can be reacted to form a gel or a hydrogel.

In one embodiment, the polymer consists essentially of a polymeric backbone comprising (or consisting essentially of) at least two monomeric repeat units B and C which are different from each other, and optionally comprising (or consisting essentially of) at least a third monomeric repeat unit A, wherein monomeric repeat unit B comprises (or consists essentially of) a silane monomeric repeat unit, and monomeric repeat unit C comprises (or consists essentially of) at least one side group which comprises (or consists essentially of) at least one optionally protected alkynyl group. This polymer can be uncrosslinked or crosslinked. This polymer, in one embodiment, can be part of a multilayer. This polymer, in one embodiment, can be reacted to form a gel or a hydrogel.

In one embodiment, the polymer consists of a polymeric backbone comprising (or consisting of) at least three monomeric repeat units A, B, and C which are different from each other, wherein monomeric repeat unit C comprises (or consists of) at least one side group which comprises (or consists of) at least one optionally protected alkynyl group. This polymer can be uncrosslinked or crosslinked. This polymer, in one embodiment, can be part of a multilayer. This polymer, in one embodiment, can be reacted to form a gel or a hydrogel.

In one embodiment, the polymer consists of a polymeric backbone comprising (or consisting of) at least two monomeric repeat units A and C which are different from each other, and optionally comprising (or consisting of) at least a third monomeric repeat unit B, wherein monomeric repeat unit A is a substituted acrylamide monomeric repeat unit, and monomeric repeat unit C comprises (or consists of) at least one side group which comprises (or consists of) at least one optionally protected alkynyl group. This polymer can be uncrosslinked or crosslinked. This polymer, in one embodiment, can be part of a multilayer. This polymer, in one embodiment, can be reacted to form a gel or a hydrogel.

In one embodiment, the polymer consists of a polymeric backbone comprising (or consisting of) at least two monomeric repeat units B and C which are different from each other, and optionally comprising (or consisting of) at least a third monomeric repeat unit A, wherein monomeric repeat unit B comprises (or consists of) a silane monomeric repeat unit, and monomeric repeat unit C comprises (or consists of) at least one side group which comprises (or consists of) at least one optionally protected alkynyl group. This polymer can be uncrosslinked or crosslinked. This polymer, in one embodiment, can be part of a multilayer. This polymer, in one embodiment, can be reacted to form a gel or a hydrogel.

In one embodiment, the polymer is represented by DMA-PMA-MAPS, copolymerized N,N-dimethylacrylamide (DMA), *3-trimethylsilanyl-prop-2-yn methacrylate* (PMA) and 3(trimethoxysilyl)-propylmethacrylate (MAPS). In one embodiment, the PMA is deprotected.

In another aspect, provided is a composition prepared by reaction of a polymer composition as described and/or claimed herein, wherein the polymer is in an unprotected form, with at least one compound. The compound can be an azide compound. The compound can be, for example, not limited by a particular molecular weight but can be a small molecule or polymer. The polymer can be a biomolecular compound. The polymer can be a synthetic polymer.

In another embodiment, provided is a composition prepared by reaction of a polymer composition as described and/or claimed herein, wherein the polymer is in an unprotected form, with at least one biomolecular compound, which optionally is a glycan compound. In another embodiment, the biomolecular compound, which optionally is a glycan compound, comprises an azide moiety.

Another aspect is an article comprising at least one substrate coated with a composition as described and/or claimed herein. In one embodiment, the article is a microarray. In another embodiment, the article can be a device for separation.

Another aspect is a method of forming the polymer composition as described and/or claimed herein, wherein the method comprises polymerizing at least one first monomer C' which provides for monomeric repeat unit C, with one or both of monomers A' and B' which provide for monomeric repeat unit A and B, respectively. The monomer C' can either directly provide the monomeric repeat unit C, or it can provide a precursor which upon a post-polymerization reaction can form the monomeric repeat unit C.

In one embodiment, the polymerizing is carried out by free radical polymerization. In another embodiment, the polymerizing is carried out with monomers A', B', and C'.

Another aspect is a method of carrying out a test, such as a binding test, wherein the method comprises exposing an article as described and/or claimed herein to a composition comprising at least one biomolecule.

In one aspect, the composition as described and/or claimed herein is crosslinked. In one aspect, the composition as described and/or claimed herein is a gel or is a hydrogel. In one embodiment, the prepared composition is a hydrogel comprising poly(alkylene glycol) polymer.

Another aspect is a method for separation comprising separating components, wherein a composition as described and/or claimed herein is used as a separation agent. Another aspect is a method for electrophoretic separation comprising electrophoretically separating components, wherein a composition as described and/or claimed herein is used as a electrophoretic sieving matrix.

Another aspect is an article as described and/or claimed herein, wherein the substrate of the article is coated with a multi-layer comprising a composition as described and/or claimed herein.

Another aspect is an article as described and/or claimed herein, wherein the multi-layer comprises at least three layers, and the surface layer comprises the composition as described and/or claimed herein.

One or more advantages in various embodiments are noted throughout the rest of this application.

BRIEF DESCRIPTION OF THE DRAWINGS

Figure 1: Chemical formula of 11 compounds: azide cynine dye (1), $\alpha$-mannose derivatives (2-9) positive (10, $\alpha$-mannose) and negative (11, $\beta$-galactose) controls.

Figure 2(a) and (b): Mean fluorescence intensity of the glycomimetics of Figure 1 (11 replicates per line) incubated with 100ng/ml of biotynilated ConA (0.943 nM) and revealed with Cy3 labeled streptavidin, (a) image of the glycomimetic microarray; (b) histogram of spot fluorescence intensity of 11 spot replicates.

Figure 3: Synthesis of the poly(DMA-PMA-MAPS) copolymer. In brackets are the molar fractions of the monomers.

Figure 4: Reaction scheme of a typical click reaction between the surface and the glycan.

Figure 5: Dependence of surface immobilization density on the concentration of the solution spotted

Figure 6(a) and (b): Fluorescence vs log([ConA]) in a sigmoidal/growth graph. Both the glycomimetics (3 and 5) were spotted at 50 $\mu$M printing concentration. The bars represent the standard deviation of each mean fluorescence. (a) is the trend of a glycan with higher affinity for ConA (0.436 nM) while (b) is the trend of a glycan with a lower affinity with the lectin (3.45 nM).

Figure 7. Separation of DNA fragments in a "click" gel (Example 2).

Figure 8. DNA microarray of different slides with multilayers of "click" functional polymers, upper showing spots and lower showing fluorescent intensity for layer one or layer three (Example 3).

DETAILED DESCRIPTION

INTRODUCTION

Microarrays are known in the art. See, for example, Muller and Roder, Microarrays, Elsevier, 2006 and Kohane, Kho, Butte, Microarrays for an Integrative Genomics, MIT Press, 2003. See also, for example, US Patent No. 8,809,071 and WO 2011/124715 which describe copolymers which can be used in microarrays.

In the lead aspect, provided is a composition comprising at least one polymer, wherein the polymer comprises a polymeric backbone comprising (or consisting essentially of, or consisting of) at least three monomeric repeat units A, B, and C which are different from each other, wherein monomeric repeat unit C comprises at least one side group which comprises at least one optionally protected alkynyl group. In some embodiments, monomeric repeat unit A may be omitted, and in some embodiments, monomeric repeat unit B may be omitted. Each of these elements is described in more detail herein below.

The polymeric backbone can be an all-carbon backbone represented by $-[CH_2-CHR]_n-$; wherein R is for the different side groups which will provide for the monomeric repeat units A, B, and C. In some embodiments, at least 90 mole%, or at least 95 mole%, or at least 99 mole% of the monomer repeat units are units A, B, and C. The different monomeric repeat units can be substantially randomly arranged in the backbone, or they can be arranged with some order. Other monomer repeat units such as D or E can be present, but in a preferred embodiment, the polymer backbone consists of or consists essentially of the repeat units A, B, and C.

The number average molecular weight can be, for example, 5,000 to 100,000, or 10,000 to 50,000.

The polymer can be purified by methods known to the person skilled in the art such as precipitation, extraction, and the like.

MONOMERIC REPEAT UNIT C, ALKYNYL GROUPS, PROTECTED AND UNPROTECTED FORMS

The monomeric repeat unit C can provide the polymer with the function of reacting with a biomolecule by Cu(I))-catalyzed of alkyne-azide cycloaddition, a method known in the art as an example of "click chemistry" rather than more conventional "nucleophilic reactions."

Apart from the Cu(I)-catalyzed 1,3-dipolar cycloaddition of alkyne and azide groups (CuAAC reaction) for forming the polymer reaction product, also another type of related reaction can be used, the so called Strain-promoted Azide-Alkyne Click Chemistry (SPAAC) reaction. The requirement of a cytotoxic copper catalyst can limit the usage of CuAAC reactions. A Copper free method is the SPAAC reaction [Jewett et al. (2010), "Cu-free click cycloaddition reactions in chemical biology," Chem. Soc. Rev. 39(4):1272]. SPAAC reactions rely on the use of strained cyclooctynes that possess a remarkably decreased activation energy in contrast to terminal alkynes and thus do not require an exogenous catalyst [Ess et al.(2008), "Transition states of strain-promoted metal-free click chemistry: 1,3-dipolar cycloadditions of phenyl azide and cyclooctynes," Org. Lett. 10: 1633]. In this embodiment, the alkyne group of the polymer is a strained cyclooctyne that can be introduced into the polymer by post modification reaction with, for example, dibenzocyclooctyne-amine (DBCO).

The SPAAC reaction can be used to form a variety of polymers and types of polymers in various applications including, for example, gels, hydrogels, and multilayers as described more hereinbelow.

In one embodiment, the optionally protected alkynyl group is an unprotected alkynyl group. More particularly, the optionally protected alkynyl group is in one embodiment an unprotected alkynyl group represented by $- C \equiv C - H$.

In another embodiment, the optionally protected alkynyl group is protected. In one embodiment, the optionally protected alkynyl group is protected by a silane group. In one embodiment, the protected alkynyl group is represented by $- C \equiv C - Si(R)_3$, wherein R is, for example, a $C_1$-$C_{12}$ alkyl group. In one embodiment, the optionally protected alkynyl group is represented by $- C \equiv C - Si(CH_3)_3$.

Monomeric repeat unit C can result from use of monomers called C'.

In addition, monomeric repeat unit C can be provided through reaction of a pre-cursor, reactive polymer. For example, a monomer can be used which is functionalized to react to prepare a polymer which has the functional groups ready to react. These functional groups (e.g., an active ester such as a succinimidyl ester) can be reacted with a multi-functional group such as propargylamine which provides the polymer with the alkyne functional groups.

MONOMERIC REPEAT UNIT A

The monomeric repeat unit A can provide the function of having the polymer absorb to the substrate surface. See, for example, US Patent No. 8,809,071 and WO 2011/124715. For example, the monomeric repeat unit can be a polymerized acrylamide moiety including, for example, a monomeric repeat unit which comprises at least one N,N-substituted acrylamide repeat unit such as polymerized dimethylacrylamide. Monsubstituted acrylamide can also be used. Monomeric repeat unit A can result from use of monomers called A'.

MONOMERIC REPEAT UNIT B

The monomeric repeat unit B can provide the function of stabilizing the absorbed film by covalently reacting with functional groups present on the surface. See, for example, US Patent No. 8,809,071 and WO 2011/124715. Monomeric repeat unit C can comprise at least one silane reactive side group. Monomeric repeat unit B can result from use of monomers called B'.

METHOD OF MAKING POLYMER

Also provided herein are methods of making polymers and the polymers which results from these methods. For example, one embodiment is a method of forming the polymer composition as described herein, wherein the method comprises polymerizing at least one first monomer C' which provides for monomeric repeat unit C, with one or both of monomers A' and B' which provide for monomeric repeat unit A and B, respectively. In one embodiment, the polymerizing is carried out by free radical polymerization. In one embodiment, the polymerizing is carried out with monomers A', B', and C'.

## METHOD OF USING THE POLYMER

One embodiment is a method of carrying out a binding test, wherein the method comprises exposing an article as described herein to a composition comprising at least biomolecule such as, for example, one or more azido-modified biomolecules. The biomolecule can bind with the article. Biomolecules include, for example, glycans, proteins, and DNA peptides. Any biomolecule which can be azide-modified can be used.

## DERIVATIZED FORM OF THE POLYMER

One embodiment is a composition prepared by reaction of a polymer composition as described herein, wherein the polymer is in an unprotected form, with at least one compound such as a biomolecule. In one embodiment, the compound is a glycan compound. Other embodiments include, for example, proteins and DNA peptides. The biomolecule such as a glycan compound can comprise an azide moiety.

The polymer can also be derivatized or crosslinked to form a crosslinked form of the polymer including a gel or hydrogel.

## ARTICLES WITH POLYMER

One embodiment is an article comprising at least one substrate coated with the compositions described herein. In a lead embodiment, the article is a microarray.

Substrates are known in the art and include, for example, glass, plastic, materials used in the semiconducting industry such as Si or $SiO_2$, and the like. Substrates can be insulators, electronic conductors, or semiconductors.

The substrates can be coated with polymer films as described herein. Film thickness can be, for example, 1 nm to 100 nm, or 2 nm to 50 nm.

## PREFERRED EMBODIMENTS AND WORKING EXAMPLES

Herein, in a preferred embodiment and in working examples, the inventor describes a novel substrate for the fabrication and screening of glycan arrays combining the high sensitivity and superior signal-to-noise ratio of polymer-coated Si-SiO_2 wafers with the immobilization by the cupper catalyzed azide/alkyne 'click' reaction[18] on a 3D coating. The inventor reports here in a preferred embodiment and working examples for the first time the synthesis and characterization of the novel *clickable polymer* and its use to form a coating on a $Si/SiO_2$ wafer for the highly sensitive detection of mono- and oligosaccharide/proteins interactions.

In this preferred embodiment and working examples, the proposed click conjugation chemistry, featuring quantitative yields, high tolerance of functional groups as well as insensitivity to solvents, fulfills many requirements for the immobilization of sugar ligands onto polymer coated supports, and it can be potentially extended to the immobilization and analysis of glycomimetic structures.

Herein, in a preferred embodiment and working examples, the inventor(s) introduce a new polymer obtained from the polymerization of N,N-dimethylacrylamide (DMA), *3-trimethylsilanyl-prop-2-yn methacrylate* (PMA) and 3(trimethoxysilyl)-propylmethacrylate (MAPS), copoly (DMA-PMA-MAPS) and describe its use in the formation of a functional coating for microarrays. The backbone of the polymer bears alkynyl side group moieties that allow binding azide-modified glycans to the surface by "Click" chemistry. This attachment mode offers a number of advantages in the immobilization of biomolecules such as glycans, such as high grafting efficiency, oriented immobilization and insensitivity to functionalities present in natural glycans. The novel surface chemistry was used to prepare microarrays substrates for fluorescence microarray on $Si/SiO_2$ slides. The higher sensitivity to the fluorescence signal provided by the novel $Si/SiO_2$ microarray substrate offers significant advantages over conventional glass slides allowing analysis at lower glycan surface density.

Eight $\alpha$-mannoside derivatives, immobilized on the polymer-modified substrate, were screened against the mannose-binding lectin Concanavalin A (Con A), using $\alpha$-mannose as the positive control and $\beta$-galactose as the negative control. The array analysis showed specific interactions of the mannosylated support with ConA with a high signal-to-noise ratio. At the highest surface densities of mannose derivatives, dissociation constants on the order of 1 nM were calculated from fluorescence microarray experiments. The surface equilibrium dissociation constant ($K_D$) of the interaction was found to depend strongly on the surface concentration of glycans. The fluorescence detection enhanced by the $Si/SiO_2$ substrates enabled the study of density dependent, binding properties of Concanavalin A even at low glycan density and to determine surface equilibrium constants in solution-like conditions.

WORKING EXAMPLES

Additional embodiments are provided in the following non-limiting working examples:

## 1. Materials and methods

### 1. 1 Materials

Trimethylsilylpropyn-1-ol, triethylamine (TEA), diethyl ether ($Et_2O$), methacryloyl chloride ($CH_2CCH_3COCl$), dry tetrahydrofuran (THF), $\alpha,\alpha'$-azoisobutyronitrile (AIBN), petroleum ether (EtP), potassium carbonate ($K_2CO_3$), copper sulphate penta-hydrate ($Cu_2SO_4 \cdot 5H_2O$), ascorbic acid, biotinylated ConcanavalinA (ConA), streptavidin-cyanine3, phosphate saline buffer (PBS), Bovin Serum Albumin (BSA), trizma base (Tris), chloridric acid (HCl), sodium chloride (NaCl), Tween 20, manganese chloride ($MnCl_2$), calcium chloride ($CaCl_2$), sodium hydroxide (NaOH), N-(2-hydroxyethyl)piperazine-N'-(2-ethanesulfonic acid) (HEPES) were purchased from Sigma Aldrich (St. Louis , MO, USA). Cyanine3 azide was purchased from Lumiprobe GmbH (Feodor-Lynnen Strasse 23, 30625 Hannover, Germany). All solvents were used as received.

Silicon oxide chips with a 100 nm thermal oxide layer were bought from Silicon Valley Microelectronics (Santa Clara, CA, USA). The glass substrates with a silicon dioxide anti-reflection layer used in some experiments were provided by ODL S.r.l. (Brembate Sopra, Bergamo, Italy). An Agilent 1200 series liquid chromatography system, (Agilent Technologies, Santa Clara, CA, USA) was used to carry out GCP. GPC columns were from Schodex (New York, NY, USA); MALLS system was purchased from Wyatt Technology (Santa Barbara, CA, USA).

### 1.2 Polymer synthesis

#### 1.2.1 Synthesis of 3-trimethylsilyl-prop-2-ynyl methacrylate (PMA)

According to Ladmiral V. and co-workers (16) 3-(trimethylsilyl)prop-2-yn-1-ol (2.31 ml, 15.6 mmol) and triethylamine (2.83 ml, 20.27 mmol) were dissolved in $Et_2O$ (20 ml) and cooled to -20°C. A solution of methacryloyl chloride (1.81 ml, 18.56 mmol) in $Et_2O$ (10 ml) was added drop wise over 1 hour. The mixture was stirred at -20°C for 30 minutes and then overnight at room temperature. Ammonium salts were removed by filtration and the volatiles were removed under reduced pressure. The yellow oil residue was purified by flash chromatography ($EtP:Et_2O=50:1$, Rf= 0.39) (2.48 g, 12.64 mmol, Yield 81%).

$^1$H-NMR (400 MHz, $CDCl_3$): $\delta$ = 0.18 (s, 9H, $Si(CH_3)_3$); 1.97 (m, 3H, $CH_3C{\equiv}CH_2$); 4.76 (s, 2H, $OCH_2$); 5.62 (m, 1H, C=C$H$H); 6.17 (m, 1H, C=CH$H$).

#### 1.2.2 Synthesis of copoly(N,N-dimethylacryiamide (DMA)-3-trimethylsilyl-prop-2-ynyl methacrylate (PMA)-3-(Trimethoxysilyl)propyl methacrylate (MAPS)).

The polymer was synthesized via a random radical polymerization in anhydrous tetrahydrofuran with a 20% w/v total monomer concentration. The DMA was filtered on aluminium oxide to remove the inhibitor. The molar fraction of the monomers DMA, PMA and MAPS was 97:2:1.

The DMA and PMA monomers were dissolved in dried tetrahydrofuran (THF) in a round-bottom flask equipped with condenser, magnetic stirring. The solution was degassed by alternating argon purges with a vacuum connection, over a 10-min period. MAPS and $\alpha,\alpha'$-Azoisobutyronitrile (this latter at 2mM final concentration) were added to the solution, which was then warmed to 65 °C and maintained at this temperature under a slightly positive pressure of argon for 2 h.

After the polymerization was completed, the solution was first diluted to 10% w/v with dry THF and the polymer precipitated by adding petroleum ether (10 times the reaction volume). The product, a white powder, was filtered on Buckner funnel and dried under vacuum at room temperature.

The protective trimethylsilyl groups were removed in water under basic condition, using $K_2CO_3$ (9mM) at pH 9. The reaction mixture was stirred at room temperature for 1h, then the polymer was dialyzed, lyophilized and the white powder obtained was stored at -20 °C.

#### 1.2.3 Polymer characterization by Gel Permeation Chromatography

The size of each polymer was characterized using Gel Permeation Chromatography in tandem with an UV-detector ($\lambda$=214nm).

A JASCO 880 PU liquid chromatography system, consisting of an isocratic pump to control mobile phase flow throughout the system connected to a JASCO UVIDEC-100-III UV detector. ChromNAV Chromatography Data System -JASCO was used to analyze the sequence of sample injection and to calculate the calibration curve of polyacrylamide standards.

The GPC setup consists of four Shodex aqueous GPC columns in series: OHpak SB-G (guard column), OHpak SB-804M HQ, OHpak SB-803 HQ, and OHpak SB-802.5 HQ. Each column is packed with a polyhydroxymethacrylate gel and connected in series with a decreasing exclusion limit. The columns were maintained at 40°C throughout each run using a thermostated column compartment.

After the polymer sample is fractionated by GPC, the sample flows into a UV-detector. The molecular weight of the polymer was obtained by using a calibration curve.

Copoly(DMA-PMA-MAPS) sample was diluted using the GPC mobile phase (GPC buffer: 100 mM NaCl, 50 mM $NaH_2PO_4$, pH 3, 10% v/v Acetonitrile) to a concentration of 2.66 mg/ml and the sample was run three times through the GPC-UV system to test for reproducibility. Each run injected 20 $\mu$L of sample to be analyzed and the flow rate through the system was held at a constant 0.3 mL/min.

*1.3 Coating of microarray slides and glass substrates with poly(DMA-PMA-MAPS)*

Poly(DMA-PMA-MAPS) was dissolved in DI water to a final concentration of 2 % w/v and then diluted 1:1 with an aqueous $(NH_4)_2SO_4$ solution at 40% of saturation. The slides were immersed into the polymer solution for 30 minutes, rinsed in DI water, dried with nitrogen flow and then cured at 80°C under vacuum for 15 minutes. Before the immersion the slide was pre-treated with oxygen plasma in a Plasma Cleaner from Harrick Plasma (Ithaca, NY, USA). The oxygen pressure was set to 1.2 Bar with a power of 29.6 W for 10 min.

*1.4 Goniometry*

Contact angle measurements were collected via the sessile drop method using a CAM200 instrument (KSV Ltd), which utilizes video capture and subsequent image analysis. Deionized water was used, and its purity was confirmed by correlating the measured surface tension based on the pendant drop shape to the literature values for pure water (72 mN/m at 25°C).

*1.5 Dual Polarization Interferometry (DPI)*

Dual polarization interferometry (DPI) measurements were conducted using an Analight Bio 200 (Farfield Group, Manchester, UK) running Analight Explorer software. A silicon oxynitride AnaChipTM surface treated with oxygen plasma was used in this study. To measure the coating thickness, the chip was inserted into the fluidic compartment of an Analight Bio 200 and a polymer solution (1% w/v in a 20% saturated ammonium sulphate) was slowly introduced into the chip channels at a flow rate of 6 $\mu$l/min for 15 minutes. The flow was then stopped, and the solution was let in contact with the surface for 30 minutes before washing the channel with water, which was injected into the channel at a flow rate of 50 $\mu$l/min.

Before each experiment, a standard calibration procedure was performed using 80 % (w/v) ethanol and MQ $H_2O$ solutions. The data were analyzed using Analight Explorer software to calculate the mass, the density and the thickness of the poly(DMA-PMA-MAPS) absorbed onto the surface.

*1.6 Microarray experiments*

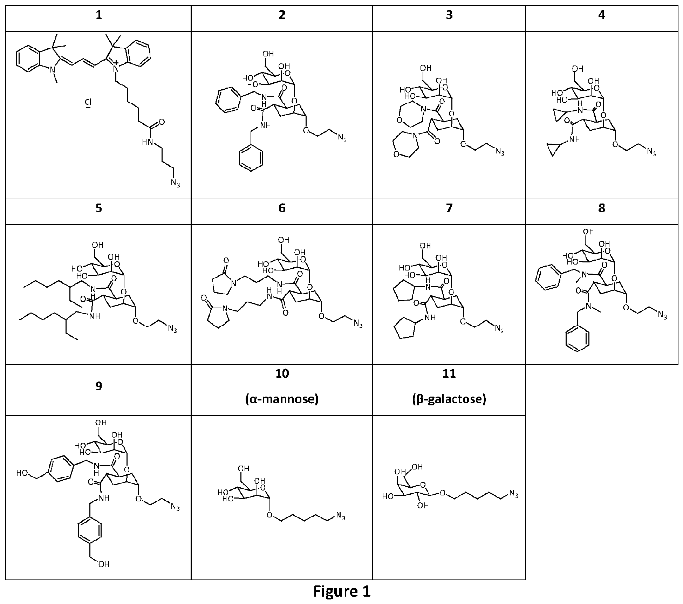

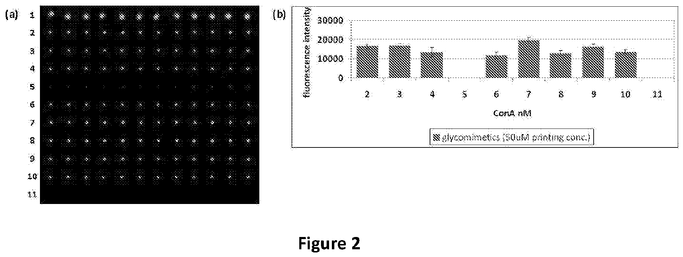

In the study of lectin-glycan interactions, an array of eight $\alpha$ -mannose derivatives carrying an azido linker was printed using a piezoelectric spotter (SciFlexArrayer S5, Scienion, Berlin Germany) on the surface of a polymer coated silicon slide. Four hundreds pL of each glycan was spotted at 10 $\mu$M or 50 $\mu$M concentration from an aqueous solution of $Cu_2SO_4 \cdot 5H_2O$ (2.5 mM) and ascorbic acid (12.5 mM). Chemical structures and entries of the glycans 2-9 used in this study are reported in Figure 1. A $\alpha$-mannoside (10) and $\beta$-galactoside (11) were used as positive and negative controls. Eleven replicates of the same glycan were spotted as shown in Figure 2a. The immobilization reaction took place during an overnight incubation in a humid chamber at room temperature. The printed slides were sequentially washed with PBS buffer for 10 minutes with DI water and dried by a nitrogen stream. The arrayed slides were then incubated with biotinylated $\alpha$-mannose-binding lectin Concanavalin A (ConA) in the lectin binding buffer (LBB, 50 mM HEPES, pH 7.4, 5mM $MnCl_2$, 5 mM $CaCl_2$) in the presence of BSA (0.2 mg/ml). After 2 hours of incubation at room temperature on a lab shaker, the slides were washed 10 minutes in washing Buffer (0.05 M Tris/HCl pH9, 0.25 M NaCl, 0.05% v/v Tween 20), rinsed in DI water and dried by a nitrogen stream. A final incubation of 1 h with 2$\mu$g/ml Cyanine3 labelled Streptavidin in PBS (Phosphate Saline Buffer) in a humid chamber at room temperature under static condition enabled the fluorescence detection of the surface bound ConA by means of a scanner (ProScanArray scanner from Perkin Elmer, Boston, MA, USA) used at 70% of laser power and 60% of photomultiplier (PMT) gain (Figure 2b). The fluorescence intensities of 11 spot replicates were confirmed by three experiments that provided the same fluorescence intensities for each glycomimetic, with a standard deviation lower than 5%.

*1.7 Determination of the surface equilibrium constant by fluorescence experiments*

The surface equilibrium constant, $K_{D,surf}$ for the interaction of eight different mannose derivatives with ConA was determined according to a method previously reported by Liang and co-workers (17). Several silicon/silicon oxide slides coated with poly(DMA-PMA-MAPS) were printed with 11 replicates of each glycan at 50 $\mu$M concentration to form an array of eight different $\alpha$-mannose derivatives. Each slide was incubated for 2 hours with a given concentration of biotinylated ConcanavalinA (ConA) (from 47.2 pM to 9.43 nM) dissolved in LBB containing 0.2mg/ml BSA.

After 1 hour of incubation with Cy-3 labeled Streptavidin (2 $\mu$g/ml) in PBS, the slides were scanned for fluorescence to evaluate the amount of ConA captured by the immobilized glycans. The Fluorescence intensities of 11 replicated spots were averaged.

The experimental conditions used during the incubation were optimized to ensure attainment of the equilibrium. The mean fluorescence intensities of the different glycans (spotted in 11 replicates) obtained from each single incubation was plotted against ConA concentration. The fluorescence values were fitted using OriginPro-8 that enables the calculation of $K_{D,surf}$ as EC50 for each glycan, depending on its affinity for ConA.

2 Results and discussion

*2.1 Design of the polymer structure and substrate selection*

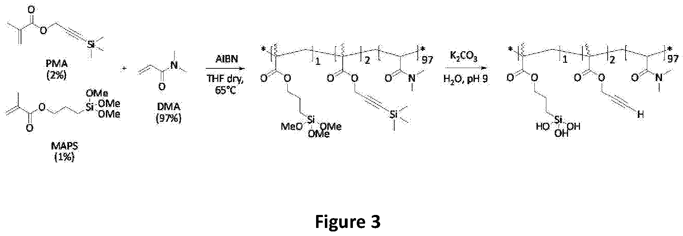

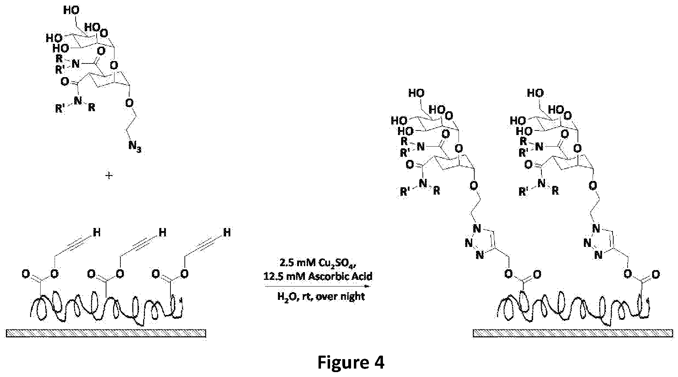

The inventor introduces a novel polymer named copoly (DMA-PMA-MAPS), obtained from the polymerization of N,N-dimethylacrylamide (DMA), *3-trimethylsilanyl-prop-2-yn methacrylate* (PMA) and 3(trimethoxysilyl)-propylmethacrylate (MAPS) (Figure 3). The GPC-MALLS analysis of poly(DMA-PMA-MAPS) indicates that the polymer has a molecular weight (Mw) of $4.2 \times 10^4$ g/mol and polydispersity of 2.6. This new polymer is different from the polymer introduced by our group to form a hydrophilic 3D coating for microarray (18-20). The novelty of this work is the introduction of an alkyne moiety which allows binding azide-modified glycans by azide alkyne Huisgen cycloaddition using a Copper (Cu) catalyst at room temperature (Figure 4). Binding glycans to the surface via click chemistry offers a number of advantages (21-23) over classical nucleophilic reactions between amino modified probes and surface active esters. From the surface point of view, the stability of an alkyne group is far higher than that of an active ester, which typically is freshly prepared right before sugar immobilization. Additionally, when building arrays of natural glycans, the selectivity of the attachment point is guaranteed, as there are no natural glycans that contain azido functions. Replacement of the N-Acryloyloxysuccinimide monomer, the chemically reactive group of the prior art "parent" polymer with PMA does not alter either the self-adsorbing properties of the polymer or its physical characteristics. The coating is prepared by "dip and rinse", by immersing the slide in an aqueous solution of the copolymer (10mg/mL) at ambient temperature followed by washing with water. The coated substrates are then cured at 80°C for 30 min. When glass-$SiO_2$ slides or Si-$SiO_2$ wafers are immersed in the copolymer solution for 30 minutes, ultrathin films of the polymer are generated. The rational behind replacing glass with Si/$SiO_2$ slides is to maximize fluorescence enhancement. As previously shown, the optical interference (OI) phenomenon induced by layers of well-defined thickness and different refractive index maximize photo-absorption of the dye molecules in the vicinity of the surface and enhance the light emitted towards the detector (24). These microarray slides display fluorescence intensity, at least, 5 times higher than that of standard glass slides.

*2.2 Surface Characterization*

*2.2.1 Contact Angle measurements*

The contact angle was measured both before and immediately after the coating deposition to monitor and quantify changes of the surface hydrophilicity resulting from the presence of a surface polymer layer. The water contact angle could not be measured on an uncoated silicon chip after 10 minutes of plasma oxygen treatment because of its extremely high hydrophilicity (i.e. complete wetting). Thanks to this characteristic, the formation of a polymer coating is immediately evident because the water droplet contact angles increase on the coated surfaces from 0° to 33° $\pm$ 0.78 °C (the obtained contact angle value is the average of five measurements each on five different coated chips).

*2.2.2 Dual Polarization Interferometry*

The coating was also characterized using dual polarization interferometry (DPI), which is an optical surface analytical technique that provides multiparametric measurements of molecules on a surface to give information on the molecular dimension (layer thickness), packing (layer refractive index, density) and surface loading (mass)(25). From the DPI analysis it was possible to characterize the polymeric coating by obtaining values of thickness, mass and density (Table 1).

### 2.2.3 Polymer binding capacity

In order to assess the density of glycans bound to the polymer coated slide, a simple experiment was carried out based on the measurement of fluorescence after spotting, immobilization and washing of an azide-modified Cyanine-3 dye (**1**, Figure 1). Following an approach described in reference 19, Cyanine 3 azide 1, was printed at concentrations ranging from 1 pM to 1 mM on copoly(DMA-PMA-NAS) coated silicon slides in 14 replicates. The slide was imaged at 543 nm with a fluorescence scanner (ProScanArray, PerkinElmer, Massachusetts, USA). After 12 hours of incubation in a dark humid chamber, the slides were washed with dimethylformamide (DMF) for 10 minutes to remove unbound molecules, dried under a nitrogen flow and imaged again to assess the binding efficiency. At a fixed laser power and photomultiplier gain (60% and 70% respectively) not all the spots could be visualized: 0.5 $\mu$M being the lowest detectable spotting concentration. Since the concentration (C) and the volume (V) of the Cy3 dye are known, the number of molecules covalently bound to the surface (Np) is the product of the number of Cy3 printed and the ratio of the pre-quench (Qpre) to post-quench (Qpost) spot intensities, where NA is Avogadro's number.

$$Np = \frac{C \cdot V \cdot N_A \cdot Qpost}{Qpre}$$

From the attachment density of the dye it was possible to estimate the distance between the molecules, which is representative of the distance between glycans.

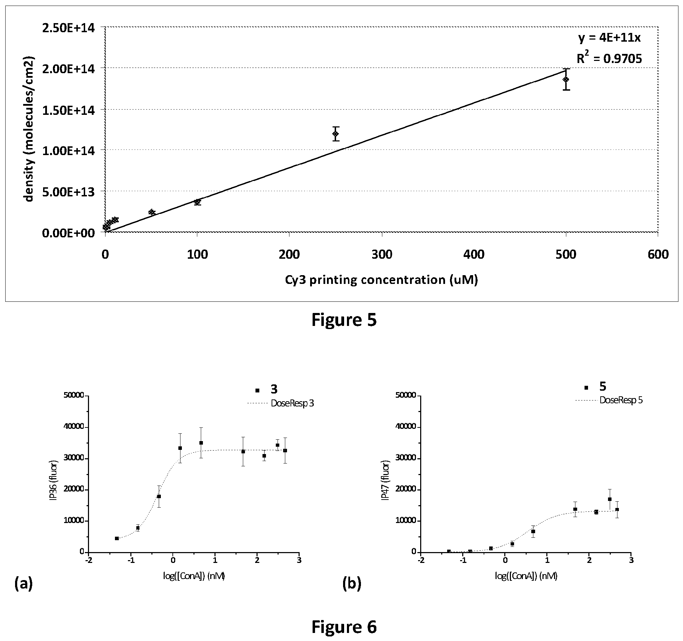

The saturation density on the polymer was found to be 3 molecules/nm$^2$. The density of bound molecule as a function of the spotted dye concentration are reported in Figure 5.

### 2.3.1 Microarray experiments

The eight $\alpha$-mannose derivatives **2-9** shown in Figure 1 were spotted on the surface of a polymer coated Si/SiO$_2$ slide at 50 $\mu$M concentration. Alpha-mannose (**10**) and $\beta$-galactose (**11**) were used as positive and negative controls, respectively, whereas the Cy3 derivative **1** (Figure 1) was used as a reference to facilitate the imaging process. Concanavalin A (ConA) was chosen in this work, due to its well characterized affinity for Mannose and Glucose derivatives (26,27).

The surface-immobilized glycans, incubated with 100 ng/ml (0.943 nM) of biotinylated ConA and detected with Cy3-labelled streptavidin, show a variable degree of fluorescent intensity (Figure 2) depending on their affinity for ConA. The interaction between $\alpha$-mannose derivatives and ConA was specific as confirmed by the lack of fluorescence on the spots of $\beta$-galactose (11), the negative control. The graph (b) of Figure 2 reports the fluorescence intensity observed for different glycan spots. Except the ligand **5,** all the mannosides of this study as well as the control **10** have similar affinities for ConA, as expected from their strong structural similarities. Differently, the ligand **5** does not seem to interact, possibly due to steric hindrance from the large, lipophilic amide groups. The analysis reported above provided only a qualitative estimate of the affinity between the $\alpha$-mannose derivatives immobilized onto the surface and the selected lectin. In order to measure the equilibrium dissociation constant ($K_D$) of the interaction a more complex experiment is required. Nine slides were spotted with 50$\mu$M and 10 $\mu$M aqueous solutions of 11 replicates of the glycomimetics **2-11** (Figure 1). The chips were incubated with ConA solutions of increasing concentration, from 47.2 pM up to 469.3 nM. By scanning the surface, a mean fluorescence value was obtained for each of the glycomimetic spot replicates. For each glycan, average values of fluorescence were plotted against ConA concentrations (logarithmic scale) and the curve was fitted as a sigmoidal/growth function. Typical curves of high (**3**) and low affinity (**5**) glycomimetics are shown in Figure 6. From these curves it was possible to extrapolate a value of EC50 (the half maximal Effective Concentration) for each molecule. EC50 refers to the ConA concentration at which half of the probes on the surface are occupied by the target. The values of EC50 reported in Table 2 represent the surface equilibrium constant $K_{D,surf}$ and provide a quantitative estimation of the affinity between the glycomimetics and the considered lectin, when the interaction occurs on a surface.

**Table 1.** Thickness, mass and density of the poly(DMA-PMA-MAPS) coating obtained from DPI analysis.

|  | Thickness (nm) | Mass (ng/mm$^2$) | Density (g/cm$^3$) |
|---|---|---|---|
| Poly-(DMA-PMA-MAPS) | 15.31 $\pm$ 3.21 | 1.98 $\pm$ 0.14 | 0.14 $\pm$ 0.04 |

**Table 2.** $K_{D,surf}$ values obtained for each glycomimetic printed at 50 $\mu$M and 10 $\mu$M concentrations.

| Glycomimetic | 50 $\mu$M *$K_{D,surf}$ (nM) | 10 $\mu$M *$K_{D,surf}$ (nM) |
|---|---|---|
| 2 | 0.26 | 1.01 |
| 3 | 0.34 | 0.79 |
| 4 | 0.67 | 1.71 |
| 5 | 5.33 | *N/A* |
| 6 | 0.88 | 1.77 |
| 7 | 0.40 | 0.98 |
| 8 | 0.34 | 0.85 |
| 9 | 0.43 | 0.75 |
| 10 | 0.90 | 1.33 |

*The values of $K_D$ were determined by incubating the slides with ConA solutions ranging in concentration from 47.2 pM to 469.3 nM. Typical dose-response curves were measured and all the data obtained were fitted with OriginPro8 using a growth/sigmoidal function fixing the parameter p=1 and the parameter A1=0.

Cited References:

(1) D. Castel, A. Pitaval, M. Debily, and X.Gidrol, Drug Discov. Today, 2006, 11, 616-622.

(2) A. Schulze, and J. Downward Nature Cell Biol. 2001, 3, 190-195.

(3) G. Ramsay, Nature Biotechnol. 1998, 16, 40-44.

(4) D. Anderson, D. Putnam, E. B. Lavik, T. A. Mahmood, R. Langer Biomaterials 2005, 26, 4892-4897.

(5) P. Kocanka, A. H. El-Sagheer and T. Brown, ChemBioChem, 2008, 9, 1280-1285

(6) H. C. Kolb, M. G. Finn and K. B. Sharpless, Angew. Chem., Int. Ed., 2001, 40, 2004-2021

(7) J. F. Lutz, Angew. Chem., Int. Ed., 2007, 46, 1018-1025

(8) H. C. Kolb and K. B. Sharpless, Drug Discovery Today, 2003, 8, 1128-1137]

(9) A. H. El-Sagheer and T. Brown, Chem. Soc. Rev., 2010, 39, 1388-1405 RSC.

(10) L. Chen, H. R. Rengifo, C. Grigoras, X. Li, Z. Li, J. Ju and J. T. Koberstein, Biomacromolecules, 2008, 9: 2345

(11) Kuzmin, A.; Poloukhtine, A.; Wolfert, M.A.; Popik, V.V., Bioconjug. Chem. 2010, 21, 2076-2085.

(12) Zhao, Y.; Liu, Y.; Lee, I.; Song, Y.; Qin, X.; Zaera, F.; Liao, J., J. Biomed. Mater. Res. 2012, 100A, 103-110.

(13) Kohn, M.; Wacker, R.; Peters, C.; Schröder, H.; Soulère, L.; Breinbauer, R.; Niemeyer, C.M.; Waldmann, H., Angew. Chem. Int. Ed. Engl. 2003, 42, 5830-5834.].

(14) W. Kusnezow, and J.D. Hoheisel, Journal of Molecular Recognition, 2003, 16, 4, 165-176.

(15) D. W. Grainger, C. H. Greef, P. Gong, M. J. Lochhead, Microarrays Methods in Molecular Biology, 2007, 381, 37-57.

(16) V. Ladmiral, G. Mantovani, G. J. Clarkson, S. Cauet, J. L. Irwin, and D. M. Haddleton, J. Am. Chem. Soc. 2006, 128, 4823-4830.

(17) P. Liang, S. Wang, and C. Wong, J. Am. Chem. Soc. 2007, 129, 11177-11184.

(18) G. Pirri, F. Damin, M. Chiari, E. Bontempi and L. E. Depero Anal. Chem. 2004, 76, 1352-1358.

(19) M. Cretich, G. Pirri, F. Damin, I. Solinas, M. Chiari Anal. Biochem. 2004, 332, 1, 67-74.

(20) M. Chiari, M. Cretich, A. Corti, F. Damin, G. Pirri, R. Longhi, Proteomics 2005, 5, 14, 3600-3603.

(21) S. Bian, J. He, K. B. Schesing, A. B. Braunschweig Small, 2012, 8, 13, 2000-2005.

(22) O. Michel, B. J. Ravoo, Langmuir 2008, 24, 12116-12118.

(23) S. Bian, J. He, K. B. Schesing, A. B. Braunschweig Small, 2012, 8, 13, 2000-2005.

(24) M. Cretich, A. Reddington, M. Monroe, M. Bagnati, F. Damin, L. Sola, M. S. Unlu, M. Chiari Biosensors and Bioelectronics 2011, 26, 3938-3943.

(25) M. Swann, L. Peel, S. Carrington, N. Freeman, Anal. Biochem., 2004, 329, 190-198.

(26) R. Loris, T. Hamelryck, J. Bouckaert, L. Wyns Biochim. Biophys. Act. 1998, 1383, 9-36.

(27) E. J. M. Van Damme, W. J. Peumans, A. Barre, P. Rouge, Crit. Rev. Plant Sci., 1998, 17, 575-692.

ADDITIONAL EMBODIMENTS:

Additional embodiments are provided including embodiments for gels and hydrogels and embodiments for multi-layers. See Working Examples 2 and 3 and supporting descriptions.

GELS AND HYDROGELS

Gels and hydrogels can be prepared by methods described herein. Gels and hydrogels are known in the art. They are cross-linked materials. Hydrogels are lightly crosslinked and swell extensively in water.

Many applications are possible with gels and hydrogels including many biochemical-oriented applications. One example of an application is a separation such as an electrophoretic separation, wherein hydrogels are used as sieving agents.

A number of hydrogels have been obtained by click chemistry reactions. They can be applied for a range of applications including, for example, drug delivery systems for the entrapment and release of pharmaceutically active proteins, and also as scaffolds for tissue engineering and repair. However, the use of click hydrogels as a sieving matrix in electrophoresis is not known.

In 2001, Sharpless has defined in *Angewandte Chemie* (Kolb et al., Angew. Chem. 2001, 113, 2056 - 2075; Angew. Chem. Int. Ed. 2001, 40, 2004 - 202) a set of criteria that a process should meet in the context of click chemistry: *"The reaction must be modular, wide in scope, give very high yields, generate only inoffensive byproducts that can be removed by nonchromatographic methods, and be stereospecific (but not necessarily enantioselective). The required process characteristics include simple reaction conditions (ideally, the process should be insensitive to oxygen and water), readily available starting materials and reagents, the use of no solvent or a solvent that is benign (such as water) or easily removed, and simple product isolation. Purification if required must be by nonchromatographic methods, such as crystallization or distillation, and the product must be stable under physiological conditions. [...] Click processes proceed rapidly to completion and also tend to be highly selective for a single product..."*. The click philosophy is based on the concepts of modularity and orthogonality: building blocks for a final target are made individually and subsequently assembled by means of click reactions. Over twenty reactions have been referred to as click reactions, one of such reactions is the Cu(I)-catalyzed cycloaddition.

In the gel and hydrogel embodiments, this type of reaction to form hydrogels can be carried out. One application is as sieving matrices for electrophoresis.

Some key elements of these embodiments include:

1) the formation of a hydrogel for DNA electophoresis by click reaction of two suitable functionalities present separately on the two *"gel forming"* components . For the definition of click reaction in the context of polymers, see Angew.Chem. Int.Ed. 2011, 50,60-62.
2) A hydrogel formed by two components where at least one of them is polymeric.
3) Optionally, both components are polymeric and multifunctional
4) Optionally, one is multifunctional and one is just functionalized at the two ends.

Copolymers described herein for click chemistry can be used to form hydrogels. For example, a first polymer can be functionalized with a click functionality such as alkyne groups. A second moiety such as a bifunctional agent or a bifunctional polymer can be functionalized with a complementary click functionality such as azide groups. The end groups of the polymer can be functionalized for the click chemistry. One or more of the components can be hydrophilic so as to provide for a hydrogel. Examples include poly(alkylene glycol) polymers and copolymers such as poly(ethylene glycol), polypropylene glycol), and copolymers of same. The degree of crosslinking can adjusted to control the degree of swelling. One exemplary polymer component which has been used to demonstrate the concept of this embodiment is described herein. It is a copolymer of dimethylacrylamide (DMA), $\gamma$-methacryloxypropyltrimethoxy silane (MAPS) and a monomer bearing alkyne functionalities, 3-(trimethylsilylpropyne) methacrylate (TMS-PMA) that, upon deprotection of the alkyne, reacts with PEG functionalized by azide moiety at both ends via Cu(I)-catalyzed 1,3-dipolar cycloaddition reaction.

The monomer bearing alkyne functionalities can be made as described herein by copolymerization. Alternatively, post modification of a functional polymer can be carried out in which, one of the monomers of a copolymer is reacted with a bifunctional molecule that bears an alkyne group. As example of this approach is given by a polymer that contains a succinymidyl active ester (NAS) that reacts with propargylamine. The result is an alkyne-functionalized polymer.

There are several ways of forming the hydrogel, the one of the example is by a reaction of poly(DMA-PMA-MAPS), the alkyne-polymer, with a second polymer that bears azide groups such as, for example, polyoxyethylene bis(azide). The length of the PEG chain can vary in a wide interval wihtout compromising its ability of cross-linking the chains of the alkyne polymer. The azido polymer can be different than PEG. Also, it can also have the same backbone of the alkyne polymer but contain azido functionalities pending from its backbone. Azido functionalities can be introduced directly in the polymerization step or be the result of a post modification process.

The Cu(I)-catalyzed 1,3-dipolar cycloaddition reaction is not the only type of click reaction that can be used to form the desired gel or hydrogel. Other examples are the reaction between thiol-functional polymer and maleimide-polymer, or thiol-polymer and alkyne-polymer catalyzed by UV in the presence of a photoinitiator, or any other type of reaction that satisfies the criteria for click chemistry.

The relative amounts of the two polymers participating in the click reaction can be adapted for the need. For example, the cross-link density and the hydrophobicity can be controlled by the ratio. For example, the weight ratio can vary from 99:1 to 1:99, or 95:5 to 5:95, or 90:10 to 10:90 with respect to either polymer. In some embodiments, for example, the majority component can be the alkyne copolymer, and the azido polymer can be the minority component. In other embodiments, the minority component can be the alkyne copolymer, and the azido polymer can be the majority component.

EXAMPLE 2

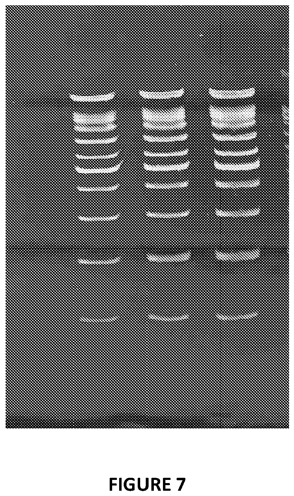

Figure 7 illustrates results from a slab gel separation of double stranded DNA fragments in a sieving matrix obtained by click chemistry reaction between copoly(DMA-MPA-MAPS) and O,O'-Bis(2-azidoethyl)polyethylene glycol catalyzed by 2.5 mM CuSO4, 12.5 mM ascorbic acid and 10 mM tris(3-hydroxypropyltriazolylmethyl)amine (THPTA). The gel was polymerized in 150 mM BisTris buffer at pH 7.2 at equimolar concentration of alkyne and azide groups. The concentration of the alkyne polymer was 10%. The DNA fragments (100 bp ladder) are stained with Sybr Green.

In example 2, copoly(DMA-NAS-MAPS) (the alkyne polymer) was synthesized as described above in Section 2.1. The polymer was dissolved at 10% w/v concentration in 150 mM BisTris-tricine buffer pH 7.2 containing 20X Sybr Green. To this solution poly(ethylene glycol) bisazide with an average Mn 1,100 from Aldrich, was added to a final concentration 10 mM (1.1% w/v). Catalysts, 2.5 mM $CuSO_4$, 10 mM tris(3-hydroxypropyltriazolylmethyl)amine (THPTA), and 12.5 mM ascorbic acid were added and the the gel was cast using a classical gel casting procedure. The solution becomes a gel in a time ranging from 30 minutes to two hours, depending on temperature. After the gel was formed, DNA (GeneRuler 100 bp) in the loading buffer (10 mM tris-HCl, sucrose and bromophenol blue) was loaded in the wells and the separation was run until the bromophenol blue contained in the DNA sample reached the end of the gel.

MULTI-LAYERS

Another embodiment for the polymers described herein is for assembly of polymer multilayer films by click chemistry. The films can be ultrathin.

Polymer multilayers obtained by click chemistry are described in, for example, Such et al., J. Am. Chem. Soc. 2006, 128, 9318-9319. A variety of substrates can be used for building up films including inorganic substrates such as glass or silicon and organic substrates such as polymers.

Herein, a composition is provided where the first layer is made by a copolymer with three important ingredients that are, for example: a susbtituted acrylamide, preferentially DMA; a silane monomer, preferentially MAPS; and an alkyne monomer or a monomer that bears a functional group that, upon rection, is tranformed into an alkyne. The role of DMA and of the silane polymer are outlined in patent application "SILANE COPOLYMERS AND USES THEREOF", EU 11714266.1 and US 2013/0115382. The simultaneous presence of the surface interacting monomer, DMA, and the surface condensing monomer, MAPS, allows to form a stable covalent coating by a simple dip and rinse approach.

On the first layer, a second layer is formed by, for example, reaction of polyoxyethylene bis(azide) with the first layer. This latter polymer can be used in large excess so to quantitatively transform the alkyne groups on the surface in azido groups. In the specific case the azido group of one end of the PEG chain reacts with alkyne groups by Cu(I)-catalyzed cycloaddition whereas the second azide, at the other end, is available for reacting with alkyne groups of a third polymer so to form the third layer.

There is large flexibility on the choice of the chemical composition of the second and third layer, as, in this case the polymer attachment is ensured by its reaction with the layer underneath and not by the combination of physi- and chemi-sorption to the surafce . Therefore, the silane condensing monomer is not required but it can optionally be present. The backbone of these polymers can be similar in composition to that of the first layer or different, the only requirement being the presence of azido or alkyne groups pending from the backbone of the polymers or located at their ends. In the formation of the third layer, polyoxyethylene bis(alkyne) can also be used.

The scope of the application is to protect a composition and its application to sensor surface modification with a functional layer so to allow covalent bonding of different ligands. The surface can be glass, silicon oxide, silicon nitrate, plastics, PDMS, gold, metal while the ligand can include a broad range of molecules such as biomolecules (proteins, DNA, peptides, glycans) or small organic molecules (drugs). Each of the three layers bind complementary functional groups. For instance, the layers 1 and 3 react with azido groups while the layer 2 reacts with alkyne groups. The coating can be made to contain the azide on the first layer, in this case, the second layer will be made to contain alkyne groups and the third, azido groups.

The rational behind building a multilayer structure in the context of a biosensor is to increase the distance bewteen the rigid substrate and the biomolecule so to reduce constraints in the conformation of the biomolecule. In addition, the orthogonal character of click chemistry allows oriented immobilization of molecules that are regioselectively modified by functional groups that are not naturally present in their chemical structure.

The thickness of the layers can be adapted, and the number of the layers can be adapted. For example, film thickness for one layer can be, for example 1 nm to 10 nm. The number of layers can be, for example, 2-100 layers, or 2-10 layers.

Example 3

In this example, three layers of alkyne and azide polymers were alternated on the surface of a microarray slide.

First Layer: silicon slides with an oxide coating of 100 nm were coated with a thin layer of copoly(DMA-PMA-MAPS). The polymer was dissolved in DI water to a final concentration of 2 % w/v and then diluted 1:1 with an aqueous $(NH_4)2SO_4$ solution at 40% of saturation. The slides were immersed into the polymer solution for 30 minutes, rinsed in deionized water, dried with nitrogen flow and then cured at 80°C under vacuum for 15 minutes. Before the immersion the slide was pre-treated with oxygen plasma in a Plasma Cleaner from Harrick Plasma (Ithaca, NY, USA). The oxygen pressure was set to 1.2 Bar with a power of 29.6 W for 10 minutes.

Second Layer: the coated slide was immersed in a solution of O,O'-Bis(2-azidoethyl)polyethylene glycol (4mM) containing 2.5 mM $CuSO_4$, 12.5 mM ascorbic acid and 10 mM tris(3-hydroxypropyltriazolylmethyl)amine (THPTA). The slide we left in this solution overnight and then rinsed extensively with water

Third layer: the slide with the two layers was immersed in a 1% w/v solution of copoly(DMA-PMA-MAPS) containing 2.5 mM CuSO4, 12.5 mM ascorbic acid and 10 mM tris(3-hydroxypropyltriazolylmethyl)amine (THPTA). The size of the PEG chain was 1000 Da . The slide was left overnight in this solution and then rinsed with water and dried exetnsively in vacuum at 80°C.

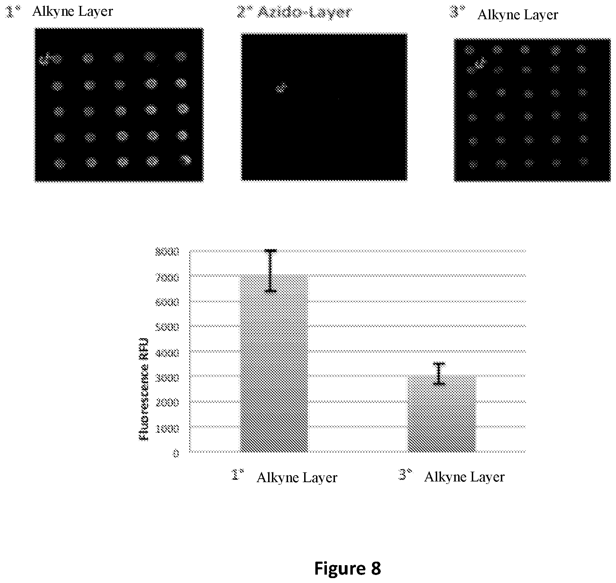

Samples of slides with the first, the second and the third layer were spotted with an azido-modified oligonucleotide (23 mer) from a solution containing the click catalysts ($CuSO_4$, ascorbic acid, THPTA). After overnight incubation the slides were washed and incubated with a solution of the fluorescently labeled complementary oligonucleotide at a 1 uM concentration for 1 hour. The slides were then rinsed with the proper buffer and imaged with a fluorescence scanner. In Figure 8a the spots of the oligonucleotide are visible in the images of the alkyne modified substrates whereas no spots are detected on the second layer as the click reaction has converted the alkyne groups almost quantitatively. These experiments prove that layers of polymers with different functional groups form on the surface. In particular, the alkyne groups on the third layer result from a click chemistry reaction between azido and alkyne polymers. The spot average fluorescence intensity detected on first and third layer is quantified in the histogram of Figure 8b. The fluorescence for the second layer was negligible as no reaction occurred.

**Claims**

1. A composition comprising at least one polymer, wherein the polymer comprises or consists essentially of or consists of a polymeric backbone comprising at least three monomeric repeat units A, B, and C which are different from each other, wherein monomeric repeat unit C comprises at least one side group which comprises at least one optionally protected alkynyl group, and
   wherein the polymer comprises an all carbon backbone.

2. The composition of claim 1, wherein monomeric repeat unit A is a substituted acrylamide monomeric repeat unit, and/or
   wherein monomeric repeat unit B comprises a silane monomeric repeat unit.

3. The composition of claim 1 or 2, wherein the optionally protected alkynyl group is an unprotected alkynyl group,

particularly wherein the optionally protected alkynyl group is an unprotected alkynyl group represented by $- C \equiv C - H$.

4. The composition of any one of claims 1-3, wherein the optionally protected alkynyl group is protected, preferably wherein the optionally protected alkynyl group is protected by a silane group, and/or wherein the optionally protected alkynyl group is protected and represented by $- C \equiv C\text{-Si(R)}_3$, wherein R is a $C_1\text{-}C_{12}$ alkyl group, preferably wherein the optionally protected alkynyl group is protected and represented by $- C \equiv C - \text{Si(CH}_3)_3$.

5. The composition of any one of claims 1-4, wherein the monomeric repeat unit A comprises at least one N,N-substituted acrylamide repeat unit and monomer B comprises at least one silane reactive side group.

6. The composition of any one of claims 1-5, wherein the monomeric repeat units are distributed randomly.

7. The composition of any one of claims 1-6, wherein the monomeric repeat unit C comprises at least one side group which comprises at least one optionally protected alkynyl group, wherein the polymer is formed by a functionalization reaction of a pre-polymer to form the optionally protected alkynyl group, and/or wherein the monomeric repeat unit C comprises at least one side group which comprises at least one optionally protected alkynyl group, wherein the optionally protected alkynyl group is represented by $-\text{NH-CH}_2\text{-C}\equiv\text{CH}$, dibenzocyclooctyne-amine, or dibenzocyclooctyne-PEG-amine, and/or wherein the optionally protected alkynyl group is a strained cyclooctyne group.

8. The composition of claim 1 or 2, wherein the polymer is represented by DMA-PMA-MAPS, copolymerized N,N-dimethylacrylamide (DMA), *3-trimethylsilanyl-prop-2-yn methacrylate* (PMA) and 3(trimethoxysilyl)-propylmethacrylate (MAPS), wherein optionally the MAPS group is deprotected.

9. A composition prepared by reaction of a polymer composition of any one of claims 1-3 or 5-8, wherein the polymer is in an unprotected form, with at least one compound, or wherein the polymer is in an unprotected form, with at least one biomolecular compound, which optionally is a glycan compound, such as a biomolecular compound, which optionally is a glycan compound, which comprises an azide moiety.

10. The composition of any one of claims 1-9, wherein the composition is cross-linked, and/or wherein the composition is a gel, and/or wherein the composition is a hydrogel, such as a hydrogel comprising poly(alkylene glycol) polymer.

11. An article comprising at least one substrate coated with a composition of any one of claims 1-10, preferably wherein the article is a microarray.

12. A method of forming the polymer composition of claim 1 or 2, wherein the method comprises polymerizing at least one first monomer C' which provides for monomeric repeat unit C, with both of monomers A' and B' which provide for monomeric repeat units A and B, respectively, preferably wherein the polymerizing is carried out by free radical polymerization.

13. A method of carrying out a binding test, wherein the method comprises exposing an article according to claim 11 or a composition according to any one of claims 1-3 or 5-10 to a composition comprising at least one biomolecule.

14. A method for separation comprising separating components, wherein the composition of any one of claims 1-10 is used as a separation agent, preferably the method is for electrophoretic separation comprising electrophoretically separating components, wherein the composition of any one of claims 1-10 is used as a electrophoretic sieving matrix.

15. The article of claim 11, wherein the substrate is coated with a multi-layer comprising the composition of any one of claims 1-10, preferably wherein the multi-layer comprises at least three layers, and the surface layer comprises the composition of any one of claims 1-10.

**Patentansprüche**

1. Zusammensetzung, umfassend mindestens ein Polymer, wobei das Polymer ein polymeres Grundgerüst umfasst oder im Wesentlichen aus einem polymeren Grundgerüst besteht oder aus einem polymeren Grundgerüst besteht, das mindestens drei monomere Wiederholungseinheiten A, B und C umfasst, die voneinander verschieden sind, wobei die monomere Wiederholungseinheit C mindestens eine Seitengruppe umfasst, die mindestens eine optional geschützte Alkinylgruppe umfasst, und
wobei das Polymer ein Vollkohlenstoff-Grundgerüst umfasst.

2. Zusammensetzung nach Anspruch 1, wobei die monomere Wiederholungseinheit A eine substituierte monomere Acrylamid-Wiederholungseinheit ist, und / oder
wobei die monomere Wiederholungseinheit B eine monomere Silan-Wiederholungseinheit umfasst.

3. Zusammensetzung nach Anspruch 1 oder 2, wobei die optional geschützte Alkinylgruppe eine ungeschützte Alkinylgruppe ist,
insbesondere wobei die optional geschützte Alkinylgruppe eine ungeschützte Alkinylgruppe ist, dargestellt durch - C ≡ C - H.

4. Zusammensetzung nach einem beliebigen der Ansprüche 1-3, wobei die optional geschützte Alkinylgruppe geschützt ist,
vorzugsweise wobei die optional geschützte Alkinylgruppe durch eine Silangruppe geschützt ist, und / oder
wobei die optional geschützte Alkinylgruppe geschützt ist und dargestellt wird durch - C ≡ C - Si(R)$_3$, wobei R eine C$_1$-C$_{12}$-Alkylgruppe ist, vorzugsweise wobei die optional geschützte Alkinylgruppe geschützt ist und dargestellt wird durch - C ≡ C - Si(CH$_3$)$_3$.

5. Zusammensetzung nach einem beliebigen der Ansprüche 1-4, wobei die monomere Wiederholungseinheit A mindestens eine N,N-substituierte Acrylamid-Wiederholungseinheit umfasst und das Monomer B mindestens eine reaktive Silanseitengruppe umfasst.

6. Zusammensetzung nach einem beliebigen der Ansprüche 1-5, wobei die monomeren Wiederholungseinheiten zufällig verteilt sind.

7. Zusammensetzung nach einem beliebigen der Ansprüche 1-6, wobei die monomere Wiederholungseinheit C mindestens eine Seitengruppe umfasst, die mindestens eine optional geschützte Alkinylgruppe umfasst, wobei das Polymer durch eine Funktionalisierungsreaktion eines Präpolymers unter Bildung der optional geschützten Alkinylgruppe gebildet wird, und / oder
wobei die monomere Wiederholungseinheit C mindestens eine Seitengruppe umfasst, die mindestens eine optional geschützte Alkinylgruppe umfasst, wobei die optional geschützte Alkinylgruppe durch -NH-CH$_2$-C≡CH, Dibenzocyclooctinamin oder Dibenzocyclooctin-PEG-amin dargestellt wird, und / oder wobei die optional geschützte Alkinylgruppe eine gespannte ("strained") Cyclooctin-Gruppe ist.

8. Zusammensetzung nach Anspruch 1 oder 2, wobei das Polymer durch DMA-PMA-MAPS, copolymerisiertes N,N-Dimethylacrylamid (DMA), *3-Trimethylsilanylprop-2-inmethacrylat* (PMA) und 3-(Trimethoxysilyl)propylmethacrylat (MAPS) dargestellt wird, wobei optional die MAPS-Gruppe entschützt ist.

9. Zusammensetzung, hergestellt durch Reaktion einer Polymerzusammensetzung nach einem beliebigen der Ansprüche 1-3 oder 5-8, wobei das Polymer in einer ungeschützten Form vorliegt, mit mindestens einer Verbindung, oder wobei das Polymer in einer ungeschützten Form vorliegt, mit mindestens einer biomolekularen Verbindung, die optional eine Glykanverbindung ist,
wie beispielsweise einer biomolekularen Verbindung, die optional eine Glykanverbindung ist, die eine Azideinheit umfasst.

10. Zusammensetzung nach einem beliebigen der Ansprüche 1-9, wobei die Zusammensetzung vernetzt ist, und / oder
wobei die Zusammensetzung ein Gel ist, und / oder
wobei die Zusammensetzung ein Hydrogel ist, wie beispielsweise ein Hydrogel, das ein Poly(alkylenglykol)-Polymer umfasst.

11. Artikel, umfassend mindestens ein Substrat, das mit einer Zusammensetzung nach einem beliebigen der Ansprüche 1-10 beschichtet ist,
vorzugsweise wobei der Artikel ein Microarray ist.

**12.** Verfahren zum Bilden der Polymerzusammensetzung nach Anspruch 1 oder 2, wobei das Verfahren das Polymerisieren von mindestens einem ersten Monomer C', das die monomere Wiederholungseinheit C bereitstellt, mit den beiden Monomeren A' und B', die die monomeren Wiederholungseinheiten A beziehungsweise B bereitstellen, umfasst, vorzugsweise wobei das Polymerisieren durch freies Radikal-Polymerisation durchgeführt wird.

**13.** Verfahren zum Durchführen eines Bindungstests, wobei das Verfahren das Aussetzen eines Artikels gemäß Anspruch 11 oder einer Zusammensetzung gemäß einem beliebigen der Ansprüche 1-3 oder 5-10 gegen eine Zusammensetzung, die mindestens ein Biomolekül umfasst, umfasst.

**14.** Verfahren zur Trennung, umfassend das Trennen von Komponenten, wobei die Zusammensetzung nach einem beliebigen der Ansprüche 1-10 als ein Trennmittel verwendet wird,
vorzugsweise ist das Verfahren für die elektrophoretische Trennung, umfassend elektrophoretische Trennkomponenten, wobei die Zusammensetzung nach einem beliebigen der Ansprüche 1-10 als eine elektrophoretische Siebmatrix verwendet wird.

**15.** Artikel nach Anspruch 11, wobei das Substrat mit einer Mehrfachschicht beschichtet ist, umfassend die Zusammensetzung nach einem beliebigen der Ansprüche 1-10,
vorzugsweise wobei die Mehrfachschicht mindestens drei Schichten umfasst und die Oberflächenschicht die Zusammensetzung nach einem beliebigen der Ansprüche 1-10 umfasst.

## Revendications

**1.** Composition comprenant au moins un polymère, dans laquelle le polymère comprend ou est constitué essentiellement de ou est constitué d'un squelette polymère comprenant au moins trois motifs répétés monomères A, B et C qui sont différents les uns des autres,
dans laquelle le motif répété monomère C comprend au moins un groupe latéral qui comprend au moins un groupe alcynyle éventuellement protégé, et
dans laquelle le polymère comprend un squelette tout en carbones.

**2.** Composition selon la revendication 1, dans laquelle le motif répété monomère A est un motif répété monomère acrylamide substitué, et/ou
dans laquelle le motif répété monomère B comprend un motif répété monomère silane.

**3.** Composition selon la revendication 1 ou 2, dans laquelle le groupe alcynyle éventuellement protégé est un groupe alcynyle non protégé,
en particulier dans laquelle le groupe alcynyle éventuellement protégé est un groupe alcynyle non protégé représenté par -C≡C-H.

**4.** Composition selon l'une quelconque des revendications 1 à 3, dans laquelle le groupe alcynyle éventuellement protégé est protégé,
de préférence dans laquelle le groupe alcynyle éventuellement protégé est protégé par un groupe silane,
et/ou
dans laquelle le groupe alcynyle éventuellement protégé est protégé et représenté par -C≡C-Si(R)$_3$, où R est un groupe alkyle en C$_1$ à C$_{12}$, de préférence dans laquelle le groupe alcynyle éventuellement protégé est protégé et représenté par -C≡C-Si(CH$_3$)$_3$.

**5.** Composition selon l'une quelconque des revendications 1 à 4, dans laquelle le motif répété monomère A comprend au moins un motif répété acrylamide N,N-substitué et le monomère B comprend au moins un groupe latéral réactif silane.

**6.** Composition selon l'une quelconque des revendications 1 à 5, dans laquelle les motifs répétés monomères sont distribués de manière aléatoire.

**7.** Composition selon l'une quelconque des revendications 1 à 6, dans laquelle le motif répété monomère C comprend au moins un groupe latéral qui comprend au moins un groupe alcynyle éventuellement protégé, dans laquelle le polymère est formé par une réaction de fonctionnalisation d'un pré-polymère pour former le groupe alcynyle éventuellement protégé, et/ou

dans laquelle le motif répété monomère C comprend au moins un groupe latéral qui comprend au moins un groupe alcynyle éventuellement protégé, où le groupe alcynyle éventuellement protégé est représenté par -NH-CH$_2$-C≡CH, la dibenzocyclooctyne-amine ou la dibenzocyclooctyne-PEG-amine et/ou

dans laquelle le groupe alcynyle éventuellement protégé est un groupe cyclooctyne contraint.

**8.** Composition selon la revendication 1 ou 2, dans laquelle le polymère est représenté par DMA-PMA-MAPS, du N,N-diméthylacrylamide copolymérisé (DMA), du *3-triméthyl-silanyl-prop-2-yne-méthacrylate* (PMA) et du 3-(triméthoxy-silyl)-propylméthacrylate (MAPS), où éventuellement le groupe MAPS est déprotégé.

**9.** Composition préparée par la réaction d'une composition polymère selon l'une quelconque des revendications 1 à 3 ou 5 à 8, dans laquelle le polymère est sous forme non protégée, ou

dans laquelle le polymère est sous une forme non protégée, avec au moins un composé biomoléculaire, qui est éventuellement un composé glycane,

tel qu'un composé biomoléculaire, qui est éventuellement un composé glycane, qui comprend un groupement azide.

**10.** Composition selon l'une quelconque des revendications 1 à 9, où la composition est réticulée,

et/ou

où la composition est un gel, et/ou

où la composition est un hydrogel, tel qu'un hydrogel comprenant un polymère poly(alkylèneglycol).

**11.** Article comprenant au moins un substrat revêtu avec une composition selon l'une quelconque des revendications 1 à 10, de préférence où l'article est un microréseau.

**12.** Procédé de formation de la composition de polymères selon la revendication 1 ou 2, où le procédé comprend la polymérisation d'au moins un premier monomère C' qui fournit le motif répété monomère C, avec les deux des monomères A' et B' qui fournissent les motifs répétés A et B monomères, respectivement, de préférence où la polymérisation est réalisée par polymérisation de radical libre.

**13.** Procédé de réalisation d'un test de liaison, où le procédé comprend l'exposition d'un article selon la revendication 11 ou d'une composition selon l'une quelconque des revendications 1 à 3 ou 5 à 10 à une composition comprenant au moins une biomolécule.

**14.** Procédé de séparation comprenant la séparation de composants, dans lequel la composition selon l'une quelconque des revendications 1 à 10 est utilisée comme agent de séparation,

de préférence le procédé est pour une séparation électrophorétique comprenant la séparation électrophorétique de composants, dans lequel la composition selon l'une quelconque des revendications 1 à 10 est utilisée en tant que matrice de tamisage électrophorétique.

**15.** Article selon la revendication 11, dans lequel le substrat est revêtu avec une multicouche comprenant la composition selon l'une quelconque des revendications 1 à 10,

de préférence dans lequel la multicouche comprend au moins trois couches, et la couche de surface comprend la composition selon l'une quelconque des revendications 1 à 10.

Figure 1

**Figure 2**

**Figure 3**

2.5 mM Cu₂SO₄,
12.5 mM Ascorbic Acid

H₂O, rt, over night

**Figure 4**

**Figure 5**

(a)

(b)

**Figure 6**

FIGURE 7

Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2012201634 A
- US 2013171461 A
- WO 100069 A
- WO 2014089247 A
- US 8809071 B
- WO 2011124715 A
- US 20130115382 A

### Non-patent literature cited in the description

- MULLER ; RODER. Microarrays. Elsevier, 2006
- KOHANE ; KHO ; BUTTE. Microarrays for an Integrative Genomics. MIT Press, 2003
- JEWETT et al. Cu-free click cycloaddition reactions in chemical biology. *Chem. Soc. Rev.,* 2010, vol. 39 (4), 1272
- ESS et al. Transition states of strain-promoted metal-free click chemistry: 1,3-dipolar cycloadditions of phenyl azide and cyclooctynes. *Org. Lett.,* 2008, vol. 10, 1633
- D. CASTEL ; A. PITAVAL ; M. DEBILY ; X.GIDROL. *Drug Discov. Today,* 2006, vol. 11, 616-622
- A. SCHULZE ; J. DOWNWARD. *Nature Cell Biol.,* 2001, vol. 3, 190-195
- G. RAMSAY. *Nature Biotechnol.,* 1998, vol. 16, 40-44
- D. ANDERSON ; D. PUTNAM ; E. B. LAVIK ; T. A. MAHMOOD ; R. LANGER. *Biomaterials,* 2005, vol. 26, 4892-4897
- P. KOCANKA ; A. H. EL-SAGHEER ; T. BROWN. *ChemBioChem,* 2008, vol. 9, 1280-1285
- H. C. KOLB ; M. G. FINN ; K. B. SHARPLESS. Int. Ed. *Angew. Chem.,* 2001, vol. 40, 2004-2021
- J. F. LUTZ. Int. Ed. *Angew. Chem.,* 2007, vol. 46, 1018-1025
- H. C. KOLB ; K. B. SHARPLESS. *Drug Discovery Today,* 2003, vol. 8, 1128-1137
- A. H. EL-SAGHEER ; T. BROWN. *Chem. Soc. Rev.,* 2010, vol. 39, 1388-1405
- L. CHEN ; H. R. RENGIFO ; C. GRIGORAS ; X. LI ; Z. LI ; J. JU ; J. T. KOBERSTEIN. *Biomacromolecules,* 2008, vol. 9, 2345
- KUZMIN, A. ; POLOUKHTINE, A. ; WOLFERT, M.A. ; POPIK, V.V. *Bioconjug. Chem.,* 2010, vol. 21, 2076-2085
- ZHAO, Y. ; LIU, Y. ; LEE, I. ; SONG, Y. ; QIN, X. ; ZAERA, F. ; LIAO, J. *J. Biomed. Mater. Res.,* 2012, vol. 100A, 103-110
- KOHN, M. ; WACKER, R. ; PETERS, C. ; SCHRÖDER, H. ; SOULÈRE, L. ; BREINBAUER, R. ; NIEMEYER, C.M. ; WALDMANN, H. *Angew. Chem. Int. Ed. Engl.,* 2003, vol. 42, 5830-5834
- W. KUSNEZOW ; J.D. HOHEISEL. *Journal of Molecular Recognition,* 2003, vol. 16 (4), 165-176
- D. W. GRAINGER ; C. H. GREEF ; P. GONG ; M. J. LOCHHEAD. *Microarrays Methods in Molecular Biology,* 2007, vol. 381, 37-57
- V. LADMIRAL ; G. MANTOVANI ; G. J. CLARKSON ; S. CAUET ; J. L. IRWIN ; D. M. HADDLETON. *J. Am. Chem. Soc.,* 2006, vol. 128, 4823-4830
- P. LIANG ; S. WANG ; C. WONG. *J. Am. Chem. Soc.,* 2007, vol. 129, 11177-11184
- G. PIRRI ; F. DAMIN ; M. CHIARI ; E. BONTEMPI ; L. E. DEPERO. *Anal. Chem.,* 2004, vol. 76, 1352-1358
- M. CRETICH ; G. PIRRI ; F. DAMIN ; I. SOLINAS ; M. CHIARI. *Anal. Biochem.,* 2004, vol. 332 (1), 67-74
- M. CHIARI ; M. CRETICH ; A. CORTI ; F. DAMIN ; G. PIRRI ; R. LONGHI. *Proteomics,* 2005, vol. 5 (14), 3600-3603
- S. BIAN ; J. HE ; K. B. SCHESING ; A. B. BRAUNSCHWEIG. *Small,* 2012, vol. 8 (13), 2000-2005
- O. MICHEL ; B. J. RAVOO. *Langmuir,* 2008, vol. 24, 12116-12118
- M. CRETICH ; A. REDDINGTON ; M. MONROE ; M. BAGNATI ; F. DAMIN ; L. SOLA ; M. S. UNLU ; M. CHIARI. *Biosensors and Bioelectronics,* 2011, vol. 26, 3938-3943
- M. SWANN ; L. PEEL ; S. CARRINGTON ; N. FREEMAN. *Anal. Biochem.,* 2004, vol. 329, 190-198
- R. LORIS ; T. HAMELRYCK ; J. BOUCKAERT ; L. WYNS. *Biochim. Biophys. Act.,* 1998, vol. 1383, 9-36
- E. J. M. VAN DAMME ; W. J. PEUMANS ; A. BARRE ; P. ROUGE. *Crit. Rev. Plant Sci.,* 1998, vol. 17, 575-692

- **KOLB et al.** *Angew. Chem.,* 2001, vol. 113, 2056-2075
- *Angew. Chem. Int. Ed.,* 2001, vol. 40, 2004-202
- *Angew.Chem. Int.Ed.,* 2011, vol. 50, 60-62
- **SUCH et al.** *J. Am. Chem. Soc.,* 2006, vol. 128, 9318-9319